# EUROPEAN PATENT SPECIFICATION

(11) **EP 0 822 186 B1**
(45) Date of publication and mention of the grant of the patent: **15.03.2000**
(21) Application number: 97117543.5
(22) Date of filing: 20.01.1995
(51) Int. Cl.: C07D 213/75

(54) **L-tert-leucine-2-pyridylamide**
L-Tertiär-leucin-2-pyridylamid
L-tertiaire-leucine-2-pyridylamide

(30) Priority: 20.01.1994 GB 9401034; 02.08.1994 GB 9415619
(43) Date of publication of application: 04.02.1998
(62) Divisional of application: 95906396.7
(73) Proprietor: BRITISH BIOTECH PHARMACEUTICALS LIMITED, Cowley Oxford, OX4 5LY (GB)
(72) Inventor: Beckett, Raymond Paul, Watlington Road, Cowley, Oxford, OX4 5LY (GB); Whittaker, Mark, Watlington Road, Cowley, Oxford, OX4 5LY (GB); Miller, Andrew, Watlington Road, Cowley, Oxford, OX4 5LY (GB); Martin, Fionna Mitchell, Watlington Road, Cowley, Oxford, OX4 5LY (GB)
(74) Representative: Walls, Alan James

(56) References cited:
- WO-A-93/20047
- WO-A-94/12169

## Description

The present invention relates to L-*tert*-leucine-2-pyridylamide, which is useful as an intermediate in the preparation of hydroxamic acid and carboxylic acid derivatives, which are inhibitors of metalloproteinases involved in tissue degradation.

Our European application no 95906396.7 (WO 95/19956) describes and claims matrix metalloproteinase inhibitor compounds of general formula I 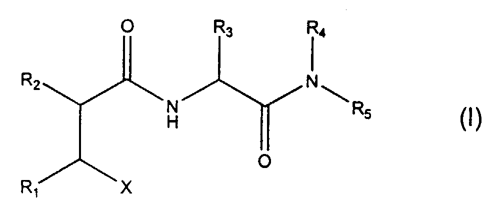 wherein
- X: is a -CO₂H or -CONHOH group;
- R₁: is hydrogen; (C₁-C₆)alkyl; (C₂-C₆)alkenyl; phenyl; substituted phenyl; phenyl (C₁-C₆)alkyl); substituted phenyl(C₁-C₆)alkyl; heterocyclyl; substituted heterocyclyl; heterocyclyl(C₁-C₆)alkyl; substituted heterocyclyl(C₁-C₆)alkyl; a group BSOₙA- wherein n is 0, 1 or 2 and B is hydrogen or a (C₁-C₆) alkyl, phenyl, substituted phenyl, heterocyclyl, (C₁-C₆)acyl, phenacyl or substituted phenacyl group, and A represents (C₁-C₆)alkyl; amino; protected amino; acylamino; OH; SH; (C₁-C₆)alkoxy; (C₁-C₆)alkylamino; di-(C₁-C₆)alkylamino; (C₁-C₆)alkylthio; aryl (C₁-C₆)alkyl; amino(C₁-C₆)alkyl; hydroxy(C₁-C₆)alkyl, mercapto(C₁-C₆)alkyl or carboxy(C₁-C₆)alkyl wherein the amino-, hydroxy-, mercapto- or carboxyl-group are optionally protected or the carboxyl- group amidated; lower alkyl substituted by carbamoyl, mono(lower alkyl)carbamoyl, di(lower alkyl)carbamoyl, di(lower alkyl)amino, or carboxy-lower alkanoylamino;
- R₂: is a (C₁-C₆)alkyl, (C₂-C₆)alkenyl, (C₂-C₆)alkynyl, phenyl(C₁₋C₆)alkyl, heteroaryl(C₁-C₆)alkyl, cycloalkyl(C₁-C₆)alkyl or cycloalkenyl(C₁-C₆) alkyl group, any one of which may be optionally substituted by one or more substituents selected from (C₁-C₆)alkyl, -O(C₁-C₆)alkyl, -S(C₁-C₆)alkyl, halo and cyano (-CN);
- R₃: is the characterising group of a natural or non-natural a amino acid in which any functional groups may be protected;
- R₄: is a phenyl or 5- or 6-membered heteroaryl ring wherein any ring nitrogen atom may be oxidised as an N-oxide, which may be optionally fused to a benzene ring or to a 5-, 6- or 7-membered heterocyclic ring, and wherein any of the rings may be optionally substituted by:
(a) one or more substituents independently selected from hydroxyl, halogen, -CN, -CO₂H, -CO₂(C₁-C₆)alkyl, -(C₁-C₆)alkyl-CO₂(C₁-C₆)alkyl,-CONH₂, -CONH(C₁-C₆)alkyl, -CON((C₁-C₆)alkyl)₂, -CHO, - CH₂OH, -(C₁-C₄)perfuoroalkyl, -O(C₁-C₆)alkyl, -S(C₁-C₆)alkyl, - SO(C₁-C₆)alkyl, -SO₂(C₁-C₆)alkyl, -NO₂, -NH₂, -NH(C₁-C₆)alkyl, - N((C₁-C₆)alkyl)₂, and -NHCO(C₁-C₆)alkyl, or
(b) a group selected from (C₁-C₆)alkyl, (C₂-C₆)alkenyl, (C₂-C₆)alkynyl, (C₃-C₈)cycloalkyl, (C₄-C₈)cycloalkenyl, phenyl, benzyl, heteroaryl or heteroarylmethyl any of which groups may be optionally substituted with one or more substituents selected from halogen, hydroxyl, amino, carboxyl, (C₁-C₄)perfluoroalkyl, (C₁-C₆)alkyl, -O(C₁-C₆)alkyl or - S(C₁-C₆)alkyl;
- R₅: is hydrogen or a (C₁-C₆)alkyl group;
or a salt, hydrate or solvate thereof, PROVIDED THAT R₄ is not 2-pyridyl or 2-thiazolyl when R₁ is hydrogen, R₂ is n-pentyl, R₃ is iso-propyl, and R₅ is hydrogen.

Examples of such compounds include those wherein R₃ is *tert*-butyl, R₄ is 2-pyridyl, and R₅ is hydrogen. The present invention relates to an intermediate for the preparation of such compounds, namely L-*tert*-leucine-2-pyridylamide, or an acid addition salt thereof.. For example, Example 23 of European application no 95906396.7 (WO 95/19956) describes the prpearation of the matrix metalloproteinase inhibitor compound R-(2,2-dimethyl-1S-pyridin-2-ylcarbamoyl-propylcarbamoyl)-5-methyl-2S-propen-2-yl-hexanohydroxamic acid, of formula: 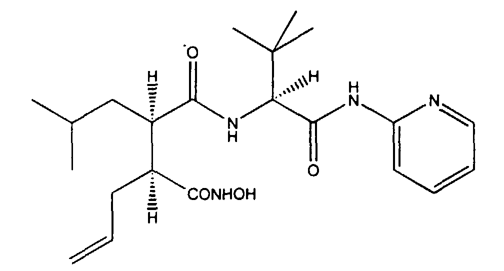 using L-*tert*-leucine-2-pyridylamide as an intermediate.

Preparations A and B below, taken together describes the preparation of L-*tert*-leucine-2-pyridylamide.

The following abbreviations have been used in Preparation A
- TLC: Thin layer chromatography
- EDC: N-Ethyl-N'-(3-dimethylaminopropyl)carbodiimide hydrochloride
¹H and ¹³C NMR spectra were recorded using a Bruker AC 250E spectrometer at 250.1 and 62.9 MHz, respectively. Elemental microanalyses were performed by CHN Analysis Ltd. (Alpha House, Countesthorpe Road, South Wigston, Leicester LE8 2PJ, UK) or Medac Ltd. (Department of Chemistry, Brunel University, Uxbridge, Middlesex UB8 3PH).

### Preparation A

### Step A

### N^{α}- Benzyloxycarbonyl-L-phenylalanine-N-phenylamide

N^{a}- Benzyloxycarbonyl-L-phenylalanine (4.95 9, 16.5 mmol) was dissolved in dichloromethane (70 ml) and the solution was cooled to 0•C and stirred during the addition of pentafluorophenol (3.35 g, 18.2 mmol), followed by EDC (3.49 g, 18.2 mmol). The mixture was allowed to warm to room temperature, stirred for a further 1 hour then cooled back to 0•C. Aniline (3.85 g, 41.4 mmol) was added dropwise and the mixture was warmed to room temperature then stirred overnight. The solution was washed twice with 1M sodium carbonate, twice with 1M hydrochloric acid and finally with brine before drying over anhydrous magnesium sulphate. The solution was filtered and evaporated to a white solid which was recrystallised from ethyl acetate-hexane. Yield: 2.57 g (41%). ¹H-NMR; δ (CDCl₃), 7.87 (1H, br s), 7.43 - 7.03 (15 H, br m), 5.62 (1H, m), 5.08 (2H, s), 4.59 (1H, s) and 3.15 (2H, s).

### STEP B:

### L-Phenylalanine-N-phenylamide

N^{α}- Benzyloxycarbonyl-L-phenylalanine-N-phenylamide (2.50 g, 6.68 mmol) was dissolved in ethanol (20 ml) and cyclohexene (5 ml) and 10% palladium on charcoal (250 mg) was added. The mixture was heated at reflux for 1 hour after which time no starting material was detectable (as indicated by TLC analysis). The catalyst was removed by filtration and the solvent evaporated to leave the title compound contaminated with residual ethanol (1.74 g). ¹H-NMR; d (CD₃OD), 7.45 (2H, m), 7.18 (7H, m), 7.04 (1H, m), 3.56 (1H, m), 3.04 (1H, dd, J = 6.4, 13.3 Hz) and 2.85 (1H, dd, J = 7.2, 13.3 Hz).

### Preparation B

### L-tert-leucine-2-pyridylamide

The title compound was prepared from N^{α}-benzyloxycarbonyl-L-*tert*-leucine by methods analogous to those described in Preparation A. ¹H-NMR; δ (CDCl₃), 8.26 (1H, m), 8.10 (1H, m), 7.74 (1H, m), 7.06 (1H, m), 3.25 (1H, s), 1.00 (9H, s).

## Claims

1. L-*tert*-leucine-2-pyridylamide or an acid addition salt thereof.

## Patentansprüche

1. L-tert-Leucin-2-pyridylamid oder Säureadditionssalz davon

## Revendications

1. L-tert-leucine-2-pyridylamide ou un de ses sels d'addition d'acide.
